Europäisches Patentamt

European Patent Office

Office européen des brevets

(19)

(11) EP 0 412 883 B1

# (12) FASCICULE DE BREVET EUROPEEN

(45) Date de publication et mention
de la délivrance du brevet:
**13.11.1996 Bulletin 1996/46**

(51) Int Cl.$^6$: **C12Q 1/68**

(21) Numéro de dépôt: **90402224.1**

(22) Date de dépôt: **02.08.1990**

(54) **Procédé rapide de détection et/ou d'identification d'une seule base sur une séquence d'acide nucléique, et ses applications**

Schnellverfahren zum Nachweis und/oder zur Identifizierung einer Einzelbase in einer Nukleinsäuresequenz und seine Verwendungen

Fast method for detection and/or identification of a single base in a nucleic acid sequence and its applications

(84) Etats contractants désignés:
**AT BE CH DE DK ES FR GB GR IT LI LU NL SE**

(30) Priorité: **11.08.1989 FR 8910802**

(43) Date de publication de la demande:
**13.02.1991 Bulletin 1991/07**

(73) Titulaire: **BERTIN & CIE**
**F-78373 Plaisir Cédex (FR)**

(72) Inventeurs:
• **Cohen, Daniel**
**F-94160 St Mande (FR)**
• **Dufau, Frédéric**
**F-78170 La Celle-St-Cloud (FR)**
• **Hache, Jean**
**F-78960 Voisins le Bretonneux (FR)**
• **Jeanpierre, Marc**
**F-75015 Paris (FR)**

• **Ginot, Frédéric**
**F-78370 Plaisir (FR)**
• **Martinez, Marie-Christine**
**F-92340 Bourg-la-Reine (FR)**
• **Roussel, Brigitte**
**F-92800 Puteaux (FR)**
• **Troton, Agnès**
**F-75010 Paris (FR)**

(74) Mandataire: **Orès, Bernard et al**
**Cabinet ORES**
**6, Avenue de Messine**
**75008 Paris (FR)**

(56) Documents cités:
**EP-A- 0 123 513      EP-A- 0 141 382**
**EP-A- 0 223 618      EP-A- 0 332 435**
**WO-A-88/05470**

## Description

La présente invention est relative à un procédé de détection et/ou d'identification d'une seule base sur une séquence d'acide nucléique, ainsi qu'à ses applications, notamment dans le diagnostic des maladies génétiques et dans le contrôle d'une hybridation.

L'hybridation d'acide nucléique a été utilisée pour étudier l'identité et établir la présence d'acides nucléiques. L'hybridation est basée sur l'appariement de bases complémentaires. Lorsque des acides nucléiques simple brin complémentaires sont incubés ensemble, lesdits acides nucléiques simple brin s'apparient pour former une molécule hybride double brin. La capacité de l'ADN simple brin ou de l'ARN à former une structure avec une séquence d'acide nucléique complémentaire est utilisée comme méthode d'analyse et de diagnostic. La disponibilité de nucléosides triphosphates radioactifs ayant une activité spécifique importante et le marquage de l'ADN au $^{32}P$ en présence d'enzymes à fonction polymérase, par exemple la $T_4$ kinase, a permis d'identifier, d'isoler et de caractériser de nombreuses séquences d'acides nucléiques d'intérêt biologique.

L'hybridation représente un critère important dans la détection de la présence de séquence d'acide nucléique particulières comme par exemple :

- dans les maladies génétiques humaines ou animales où une modification héréditaire du patrimoine génétique a des conséquences morbides (par insertion, délétion ou mutation ponctuelle d'une séquence particulière) ;
- dans les maladies cancéreuses, où des réarrangements de l'ADN génomique sont observés ;
- au cours des infections où l'on décèle la présence de génomes étrangers tels que celui des microorganismes (bactéries, champignons et virus par exemple) ;
- pour l'identification des individus en général :

  . en médecine légale (paternité, filiation par exemple),
  . dans le domaine agroalimentaire (plantes, contrôle sanitaire par exemple).

Cependant, l'hybridation comme outil de diagnostic peut être limitée par la difficulté de sa mise en oeuvre (techniques lourdes) ou par l'absence de spécificité de l'hybridation (protocole opératoire).

En effet, l'étude chimique de l'hybridation a mis en évidence l'influence de la concentration de chacun des brins d'acides nucléiques impliqués, de leurs longueurs, de leurs compositions en bases, de la température, du pH, de la force ionique, de la viscosité du milieu.

La température notamment, est critique et doit rester inférieure à la température de fusion (Tm : température à laquelle 50 % des séquences sont sous forme double brin). En solution, la température optimale d'hybridation est 25°C en dessous de la Tm pour une sonde de 150 nucléotides et légèrement plus basse pour les sondes plus courtes.

Ainsi lorsque l'on veut détecter une mutation portant sur une seule base, on peut généralement utiliser, selon les cas, deux types de sondes : des sondes d'acide nucléique dites longues, supérieures à 150 nucléotides en général, ou des sondes d'acide nucléique dites courtes entre 17 et 24 nucléotides en général. Si la mutation intervient dans un site reconnu spécifiquement par une enzyme dite de restriction, on peut utiliser la technique de Southern : celle-ci comporte les étapes d'isolement de l'ADN, de digestion par l'enzyme de restriction, d'électrophorèse sur gel, de transfert sur une membrane et d'hybridation à l'aide d'une sonde longue intéressant la région de la mutation ; après lavage et autoradiographie, l'analyse des tailles des fragments obtenus permet d'infirmer ou confirmer la présence de la mutation. Le procédé très lourd nécessite que la mutation intéresse un site de restriction. Si ce n'est pas le cas, on peut synthétiser une sonde courte oligonucléotidique de 17 à 24 nucléotides dont le centre coïncide avec la mutation que l'on veut détecter. En choisissant des conditions appropriées d'hybridation et de rinçage (spécifique de chaque système), on ne peut obtenir une hybridation à l'aide de l'oligonucléotide marquée qu'en cas d'homologie parfaite (une seule différence nucléotidique, notamment à l'endroit de la mutation, entraîne la déstabilisation de l'hybridation).

La Demande de Brevet EP 141 382 décrit un procédé d'autoradiographie, pour l'obtention d'une information permettant la localisation de substances marquées radioactivement, révélées sur un support solide. Cette méthode est en particulier applicable aux méthodes de séquençage de l'ADN, utilisant l'autoradiographie.

La Demande Internationale PCT WO 88/05470 est relative à des ADN polymérases T7 et à leurs applications dans le séquençage de l'ADN.

L'ADN polymérase décrite dans cette Demande Internationale PCT, est capable d'incorporer, de manière continue, de nombreux nucléotides (processivité supérieure à 500 bases), en utilisant le même système amorce-matrice, peut être utilisée avec des amorces de petites dimensions (10 bases ou moins, de préférence 4-20), permet l'incorporation d'analogues de nucléotides et ne présente pas d'activité nucléasique.

Cependant, ces différentes méthodes présentent un certain nombre d'inconvénients:

- conditions de température difficiles à maîtriser, pour obtenir une hybridation appropriée ;
- éventuellement présence obligatoire d'un site de restriction ;
- immobilisation de l'acide nucléique sur membrane (Southern blot).

Le Brevet Américain AMERSHAM n° 4 656 127, a permis de pallier certains de ces inconvénients, notamment en ce qu'il permet de détecter une mutation présente à un endroit ne présentant pas de site de clivage par une enzyme de restriction.

Ce Brevet Américain n° 4 656 127, décrit une méthode de détection de la mutation d'une base nucléotique spécifique dans un fragment d'acide nucléique (ADN ou ARN) cible par :

(a) hybridation d'une sonde avec la séquence cible pour former un hybride d'acide nucléique, dans lequel une extrémité de la sonde est hybridée de manière adjacente à la base nucléotidique spécifique ;
(b) mélange de l'hybride avec un dérivé nucléotidique dans des conditions appropriées à l'élongation de la sonde, de manière à permettre la jonction dérivé nucléotidique - extrémité de la sonde, seulement si la base spécifique dans la séquence cible est (ou n'est pas) la mutation à détecter, une sonde associée audit dérivé nucléotidique étant résistante à une digestion dans des conditions particulières ;
(c) digestion de l'hybride par une exonucléase dans des conditions telles que le fragment double brin est progressivement digéré à partir de l'extrémité de la sonde à moins que ladite extrémité ait été mise en jonction avec ledit dérivé nucléotidique ;
(d) élimination des portions de la sonde qui ne sont plus hybridées à la chaîne d'acide nucléique ;
(e) et détection d'une mutation de la base nucléotidique spécifique dans la séquence cible par détection de la présence ou de l'absence de la sonde après digestion.

Ce procédé implique en particulier en plus de la sonde, l'utilisation d'un dérivé nucléotidique ayant des propriétés particulières et comporte de ce fait encore de nombreuses étapes. De plus, il nécessite, pour sa réalisation, une immobilisation de l'acide nucléique sur une membrane et également un marquage (de la sonde ou du dérivé nucléotidique).

La présente invention s'est en conséquence, donné pour but de pourvoir à un procédé d'identification d'une base nucléotidique spécifique, aisé et rapide à mettre en oeuvre, qui répond mieux aux nécessités de la pratique que les procédés de l'Art antérieur, notamment en ce que le procédé conforme à l'invention ne nécessite pas un protocole opératoire complexe, c'est-à-dire ne nécessite ni immobilisation de l'ADN, ni marquage de la sonde, et s'applique à la détection de séquences d'acide nucléique particulières, notamment dans les maladies génétiques, les maladies cancéreuses, les infections, l'identification d'individus humains, animaux ou végétaux.

La présente invention a pour objet un procédé de détection et/ou d'identification d'une base nucléotidique spécifique dans une séquence d'acide nucléique cible, du type comprenant une étape d'hybridation de la séquence cible sur laquelle se trouve la base à identifier avec une séquence nucléotidique sonde de longueur suffisante pour permettre une hybridation correcte, quelle que soit la température de réaction, une étape de synthèse du brin complémentaire de l'hybride obtenu -ladite séquence nucléotidique sonde servant d'amorce-, en présence d'une polymérase sans action exonucléase 3'5', et une étape de détection de la base à identifier par tout moyen approprié,
lequel procédé étant caractérisé :

- en ce que ladite séquence nucléotidique, servant d'amorce, s'hybride avec la séquence cible, de manière à ce que son extrémité 3' soit adjacente à la base nucléotidique spécifique à détecter et/ou à identifier ; et
- en ce que la synthèse dudit brin complémentaire de l'hybride est réalisée en présence d'au moins une base nucléotidique bloquante choisie parmi les didésoxynucléotides et en l'absence de dNTP, laquelle base nucléotidique bloquante, lorsqu'elle est incorporée, est détectée et permet d'identifier la base nucléotidique spécifique complémentaire présente sur la séquence cible à analyser.

On entend par nucléotide, au sens de la présente invention, aussi bien un oligonucléotide, qui comprend de 10 à 50 bases qu'un nucléotide pouvant comprendre plus de cent bases.

Selon un mode de mise en oeuvre avantageux dudit procédé, lesdites bases nucléotidiques bloquantes sont marquées de manière appropriée notamment par un marqueur choisi dans le groupe qui comprend des substances radioactives, des enzymes, des produits chimiques chromophores fluorescents ou chimioluminescents et des anticorps appropriés.

Selon une disposition avantageuse de ce mode de mise en oeuvre, le marqueur est identique ou différent pour chacune des bases nucléotidiques bloquantes.

Selon une modalité de cette disposition, lorsque les quatre bases bloquantes sont marquées à l'aide de marqueurs différents, la détection des quatre nucléotides bloquants est avantageusement simultanée.

Selon un autre mode de mise en oeuvre avantageux dudit procédé, lorsque les quatre bases bloquantes sont marquées de manière identique ou bien lorsqu'elles ne sont pas marquées, la détection des quatre nucléotides bloquants est réalisée successivement et/ou séparément.

Selon une disposition avantageuse de ce mode de mise en oeuvre, on détecte, de manière appropriée, le pyrophosphate formé lors de la réaction de polymérisation.

En effet, la réaction de polymérisation génère la production d'un pyrophosphate, comme suit :

$$\text{matrice - amorce} + \text{dNTP} \rightarrow$$
$$\text{matrice - (amorce} + \text{dNMP)} + \text{PP}_i.$$

En mesurant le pyrophosphate dans chaque tube de réaction, il est possible de déterminer pour laquelle des bases une réaction de polymérisation s'est produite.

Selon une autre disposition avantageuse de ce mode de mise en oeuvre, on détecte chaque base nucléotidique marquée.

En effet, chaque tube de réaction contient, en ce cas, une seule base nucléotidique marquée, les trois autres ne l'étant pas ; la mesure de la base marquée (par fluorescence, radioactivité...) permet de déterminer pour laquelle des bases une réaction de polymérisation s'est produite, les bases marquées non incorporées étant éliminées par lavage.

Le procédé conforme à l'invention a notamment l'avantage de permettre de définir les conditions opératoires, indépendamment de la base nucléotidique à identifier et de ne pas nécessiter d'immobilisation de l'acide nucléique sur une membrane.

La présente invention a également pour objet un kit ou coffret de diagnostic, prêt à l'emploi, pour la mise en oeuvre du procédé conforme à l'invention, caractérisé en ce qu'il comprend outre les quantités convenables de réactifs et tampons adaptés à la mise en oeuvre du procédé :

- des quantités appropriées d'un nucléotide, servant d'amorce, capable de s'hybrider avec la séquence cible de manière à ce que son extrémité 3' soit adjacente à la base nucléotidique spécifique à détecter ;
- des quantités appropriées de quatre bases nucléotidiques bloquantes choisies parmi les didésoxynucléotides (ddTTP, ddGTP, ddATP, ddCTP), de manière à être incorporables dans le produit d'extension de l'amorce tout en bloquant l'élongation dudit produit d'extension ; et
- des quantités appropriées d'une polymérase sans action exonucléase 3'5'.

Selon un mode de réalisation avantageux dudit kit ou coffret, les bases nucléotidiques modifiées sont marquées de manière appropriée notamment par un marqueur choisi dans le groupe qui comprend des substances radioactives, des enzymes, des produits chimiques chromophores fluorescents ou chimioluminescents et des anticorps appropriés.

Selon un autre mode de réalisation avantageux dudit kit ou coffret, il comprend, en outre, des réactifs appropriés pour le dosage du pyrophosphate.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de mise en oeuvre du procédé objet de la présente invention.

Il doit être bien entendu, toutefois, que ces exemples sont donnés uniquement à titre d'illustration de l'objet de l'invention, dont ils ne constituent en aucune manière une limitation.

Exemple 1 : diagnostic de la drépanocytose par le procédé conforme à l'invention (mesure des didésoxynucléotides fluorescents).

La drépanocytose ou anémie falciforme est due à une mutation dans l'un des exons du gène β codant pour la chaîne β de l'hémoglobine (Hb). Cette mutation consistant en une substitution d'une adénine (A) par une thymine (T), modifie le codon GAG, traduit en un acide glutamique (en position G) dans l'Hb normale, en codon GTG, traduit en une valine dans l'Hb anormale (HbS). L' ADN qui a été utilisé dans cet exemple correspond à une séquence d'ADN amplifié simple-brin.

a) Blocage des amorces d'amplification.

\* mélanger dans un tube de microcentrifugation, pour chaque ADN matrice :

- 100 ng d'ADN (simple-brin amplifié)
- 7 μl de tampon Sequenase 5X (= Tris-HCl pH 7,5, 200 mM ; NaCl, 250 mM ; MgCl$_2$, 100 mM)
- H$_2$O qsp 22 μl

\* agiter à l'aide d'un vortex et centrifuger rapidement
\* incuber au bain-marie à 95°C pendant 2 minutes.
\* enlever vite et placer au bain-marie à 37°C pendant 10 minutes.
\* préparer le tampon de réaction pour un échantillon d'ADN, constitué de :

- 2,5 μl DTT 0,1 M
- 1 μl d'un mélange de ddNTP,dilué au 1/400 (ddTTP : 112 μM ; ddGTP : 1,12 μM ; ddATP : 3,36 μM ; ddCTP : 8,96 μM) ; et
- H$_2$O qsp 6,5 μl
- Sequénase 1 μl (3 unités)

\* enlever les tubes du bain-marie et centrifuger rapidement,
\* ajouter le tampon de réaction et mélanger,
\* placer au bain-marie à 37°C, pendant 5 minutes,
\* enlever les tubes et les placer dans la glace.

b) Elimination des didésoxynucléotides froids en excès

On utilise les systèmes de microséparation Centricon® 3 ou 10 (Amicon) qui, par filtration sur membrane accélérée par centrifugation, permettent de retenir des espèces moléculaires de poids moléculaire supérieur à 3 000 ou 10 000 Daltons (par exemple: un nucléotide correspond à 330 D et un oligonucléotide de synthèse de 20 meres correspond à 6 600 D).

- disposer le volume réactionnel issu de a) dans un "Centricon" 3 ou 10 et diluer si nécessaire,
- centrifuger à moins de 5 000 g en suivant les instructions du fabricant, de façon à récupérer un volume minimal,
- inverser le système Centricon®,
- centrifuger pour récupérer le volume réactionnel,
- ramener le volume obtenu à 12 µl.

c) Microséquençage (procédé conforme à l'invention)

* Pour chaque tube d'ADN, ajouter aux 12 µl :

  - 15 ng d'oligonucléotide 5' CATGGTGCACCTGACTCCTG 3' (OA), correspondant à la séquence s'arrêtant à la base adjacente à la position de la mutation
  - 7 µl de Tampon Sequénase 5X tel que défini en a) ci-dessus et procéder comme dans a) ; puis

* préparer le tampon de réaction constitué pour chaque échantillon de :

  - 2,5 µl DTT 0,1 M
  - 1 µl d'une dilution au 1/400ème d'un mélange de ddNTP fluorescents (ddTTP* : 112µM ; ddGTP* : 1,12µM ; ddATP* : 3,36µM ; ddCTP* : 8,96µM). Les didésoxynucléotides fluorescents sont vendus par Du Pont (Genesis® 2000 DNA Analysis System)
  - H$_2$O qsp 6,5 µl
  - Sequenase 1 µl (3 unités)

* enlever les tubes du bain-marie, centrifuger rapidement,
* ajouter le tampon de réaction, mélanger,
* placer au bain-marie à 37°C, pendant 5 mns,
* enlever les tubes et placer les dans la glace.

d) Lavage des didésoxynucléotides fluorescents en excès :

* passer les échantillons sur colonne de Sephadex® G50,
* récupérer les éluats.

e) Détection

La détection se fait pour chaque échantillon après migration électrophorétique et excitation par une source telle qu'un laser. Le signal est analysé par un fluoromètre.

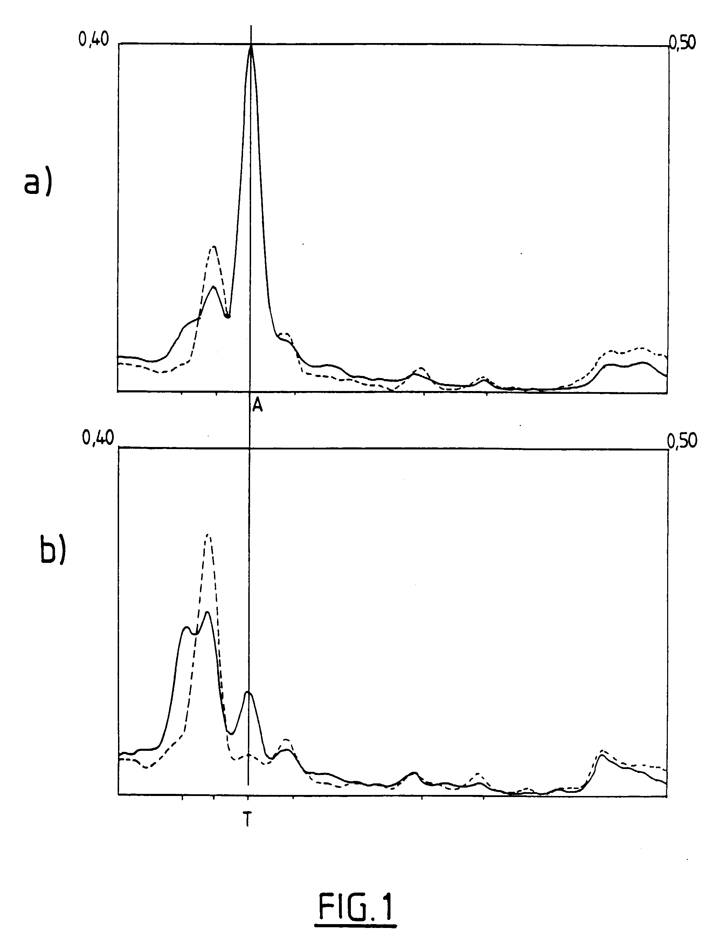

On obtient les courbes a (témoin) et b (malade), comme visible sur la figure 1, qui permet de détecter au niveau de la position correspondant à une éventuelle mutation, l'incorporation d'un ddATP en a, pour l'individu sain et pour l'individu malade homozygote en b, l'incorporation d'un ddTTP.

**Exemple 2 : Microséquençage d'une base sur une séquence d'acide nucléique (ADN du bacteriophage M13 mp8).**

a) Matériel de départ

* ADN simple brin de bacteriophage M13mp8
* Oligonucléotide de synthèse dit Primer Universel de séquence : 3'TGACCGGCAGCAAAATG5'

La première base incorporée sur l'amorce dans le sens 5'→3' est un G.

b) Protocole

Le protocole est équivalent au protocole de détection de mutation ponctuelle à partir de l'étape c de l'exemple 1. Les étapes a et b sont inutiles, 1' ADN du phage M13 étant non contaminé par des oligonucléotides.

En variante, le protocole est le suivant :

- 3 µg d'A.D.N. simple-brin M13
- 15 ng d'oligonucléotide primer universel
- 7 µl de Tampon 5X Sequenase
- H$_2$O qsp 22 µl

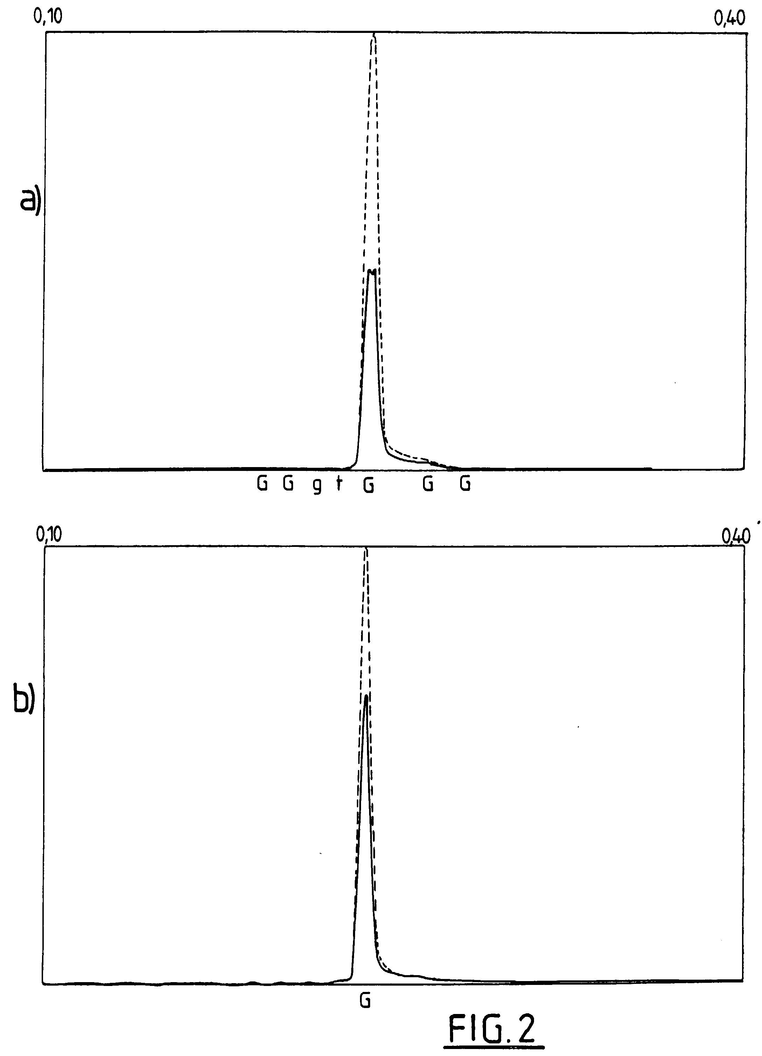

La détection révèle l'incorporation sur le primer d'un dideoxy GTP, ce qui correspond au résultat attendu, comme visible sur la figure 2, qui montre les résultats obtenus avec deux dilutions différentes des ddNTP (1/200 (a) et 1/400ème (b)).

**Revendications**

1. Procédé de détection et/ou d'identification d'une base nucléotidique spécifique dans une séquence d'acide nucléique cible, du type comprenant une étape d'hybridation de la séquence cible sur laquelle se trouve la base à identifier avec une séquence nucléotidique sonde de longueur suffisante pour permettre une hybridation correcte, quelle que soit la température de réaction, une étape de synthèse du brin complémentaire de l'hybride obtenu -ladite séquence nucléotidique sonde servant d'amorce-, en présence d'une polymérase sans action exonucléase 3'5', et une étape de détection de la base à identifier par tout moyen approprié, lequel procédé étant caractérisé :

   - en ce que ladite séquence nucléotidique, servant d'amorce, s'hybride avec la séquence cible, de manière à ce que son extrémité 3' soit adjacente à la base nucléotidique spécifique à détecter et/ou à identifier ; et
   - en ce que la synthèse dudit brin complémentaire de l'hybride est réalisée en présence d'au moins une base nucléotidique bloquante choisie parmi les didésoxynucléotides et en l'absence de dNTP, laquelle base nucléotidique bloquante, lorsqu'elle est incorporée, est détectée et permet d'identifier la base nucléotidique spécifique complémentaire présente sur la séquence cible à analyser.

2. Procédé selon la revendication 1, caractérisé en ce que lesdites bases nucléotidiques bloquantes sont marquées de manière appropriée, notamment à l'aide d'un marqueur choisi dans le groupe qui comprend des substances radioactives, des enzymes, des produits chimiques chromophores fluorescents ou chimioluminescents et des anticorps appropriés.

3. Procédé selon la revendication 2, caractérisé en ce que le marqueur est identique ou différent pour chacune des bases nucléotidiques bloquantes.

4. Procédé selon la revendication 3, caractérisé en ce que lorsque les quatre bases bloquantes sont marquées à l'aide de marqueurs différents, la détection des quatre nucléotides bloquants est avantageusement simultanée.

5. Procédé selon l'une quelconque des revendications 1 à 3, caractérisé en ce que lorsque les quatre bases bloquantes sont marquées de manière identique ou bien lorsqu'elles ne sont pas marquées, la détection des quatre nucléotides bloquants est réalisée successivement et/ou séparément.

6. Procédé selon la revendication 5, caractérisé en ce qu'on dose, de manière appropriée, le pyrophosphate formé lors de la réaction de polymérisation, ledit dosage de pyrophosphate permettant de déterminer pour laquelle des bases une réaction de polymérisation s'est produite.

7. Procédé selon la revendication 5, caractérisé en ce qu'on détecte chaque base nucléotidique bloquante marquée.

8. Kit ou coffret de diagnostic, prêt à l'emploi, pour la mise en oeuvre du procédé selon l'une quelconque des revendications 1 à 7, caractérisé en ce qu'il comprend outre les quantités convenables de réactifs et tampons adaptés à la mise en oeuvre du procédé :

- des quantités appropriées d'un nucléotide, servant d'amorce, capable de s'hybrider avec la séquence cible de manière à ce que son extrémité 3' soit adjacente à la base nucléotidique spécifique à détecter ;
- des quantités appropriées de quatre bases nucléotidiques bloquantes choisies parmi les didésoxynucléotides, de manière à être incorporables dans le produit d'extension de l'amorce tout en bloquant l'élongation dudit produit d'extension ; et
- des quantités appropriées d'une polymérase sans action exonucléase 3'5'.

9. Kit ou coffret selon la revendication 8, caractérisé en ce que les bases nucléotidiques bloquantes sont marquées de manière appropriée, notamment à l'aide d'un marqueur choisi dans le groupe qui comprend des substances radioactives, des enzymes, des produits chimiques chromophores fluorescents ou chimioluminescents et des anticorps appropriés.

10. Kit ou coffret selon la revendication 8 ou la revendication 9, caractérisé en ce qu'il comprend, en outre, des réactifs appropriés pour le dosage du pyrophosphate.

11. Application du procédé selon l'une quelconque des revendications 1 à 7, à la détection de la présence de séquences d'acides nucléiques particulières associées à des maladies, telles que maladies génétiques ou maladies cancéreuses, à des infections, ou propres à permettre l'identification d'individus humains, animaux ou végétaux.

**Patentansprüche**

1. Verfahren zum Nachweis und/oder zur Identifikation einer spezifischen Nukleotidbase in einer Zielnukleinsäuresequenz, umfassend eine Stufe der Hybridisierung der Zielsequenz, in der sich die zu identifizierende Base befindet, mit einer Sondennukleotidsequenz einer ausreichenden Länge, um eine korrekte Hybridisierung unabhängig von der Reaktionstemperatur zu erlauben, eine Stufe der Synthese des Komplementärstranges des erhaltenen Hybrids - wobei die Sondennukleotidsequenz als Startersequenz dient - in Gegenwart einer Polymerase ohne 3'5'-Exonukleasewirkung und eine Stufe des Nachweises der zu identifizierenden Base durch jedes geeignete Mittel, dadurch **gekennzeichnet**,

- daß die als Startersequenz dienende Nukleotidsequenz mit der Zielsequenz so hybridisiert, daß deren 3'-Ende der spezifischen, nachzuweisenden und/oder zu identifizierenden Nukleotidbase benachbart ist; und
- daß die Synthese des dem Hybrid komplementären Stranges in Gegenwart mindestens einer blockierenden Nukleotidbase, ausgewählt aus den Didesoxynukleotiden, und in Abwesenheit von dNTP durchgeführt wird, wobei die blockierende Nukleotidbase bei Einbau nachgewiesen wird, und die Identifikation der spezifischen, komplementären, in der zu analysierenden Zielsequenz vorhandenen Nukleotidbase erlaubt.

2. Verfahren nach Anspruch 1, dadurch **gekennzeichnet**, daß die blockierenden Nukleotidbasen in geeigneter Weise, insbesondere mit Hilfe eines Markers, ausgewählt aus der Gruppe, umfassend radioaktive Substanzen, Enzyme, chemische chromophore, fluoreszierende oder chemilumineszierende Produkte und geeignete Antikörper, markiert werden.

3. Verfahren nach Anspruch 2, dadurch **gekennzeichnet**, daß der Marker für jede der blockierenden Nukleotidbasen identisch oder unterschiedlich ist.

4. Verfahren nach Anspruch 3, dadurch **gekennzeichnet**, daß, wenn die vier blockierenden Basen mit Hilfe von unterschiedlichen Markern markiert werden, der Nachweis der vier blockierenden Nukleotide vorteilhafterweise gleichzeitig vorgenommen wird.

5. Verfahren nach einem der Ansprüche 1 bis 3, dadurch **gekennzeichnet**, daß, wenn die vier blockierenden Basen auf identische Weise markiert werden oder auch wenn sie nicht markiert werden, die Detektion der vier blockierenden Nukleotide aufeinanderfolgend und/oder getrennt durchgeführt wird.

6. Verfahren nach Anspruch 5, dadurch **gekennzeichnet**, daß man in geeigneter Weise das während der Polymerisationsreaktion gebildete Pyrophosphat bestimmt, wobei die Bestimmung von Pyrophosphat die Bestimmung erlaubt, für welche der Basen eine Polymerisationsreaktion stattgefunden hat.

7. Verfahren nach Anspruch 5, dadurch **gekennzeichnet**, daß man jede blockierende markierte Nukleotidbase nachweist.

8. Gebrauchsfertiges Kit oder Diagnosereagenszusammenstellung zur Durchführung des Verfahrens nach einem der Ansprüche 1 bis 7, dadurch **gekennzeichnet**, daß es außer geeigneten Mengen an Reagentien und Puffern, die für die Durchführung des Verfahrens geeignet sind,

- geeignete Mengen eines Nukleotids, das als Startersequenz dient, mit der Fähigkeit, mit der Zielsequenz so zu hybridisieren, daß deren 3'-Ende der spezifisch nachzuweisenden Nukleotidbase benachbart ist;
- geeignete Mengen der vier blockierenden Nukleotidbasen, ausgewählt aus den Didesoxynukleotiden, so daß sie in das Extensionsprodukt des Starters eingebaut werden können, während vollständig die Verlängerung des Extensionsprodukts blockiert wird; und

- geeignete Mengen einer Polymerase ohne 3'5'-Exonukleasewirkung, umfaßt.

9. Kit oder Reagenszusammenstellung nach Anspruch 8, dadurch **gekennzeichnet**, daß die blockierenden Nukleotidbasen in geeigneter Weise, insbesondere mit Hilfe eines Markers, ausgewählt aus der Gruppe, umfassend radioaktive Substanzen, Enzyme, chemische chromophore, fluoreszierende oder chemilumineszierende Produkte und geeignete Antikörper, ausgewählt sind.

10. Kit oder Reagenszusammenstellung nach Anspruch 8 oder 9, dadurch **gekennzeichnet**, daß es weiterhin geeignete Reagentien für die Bestimmung von Pyrophosphat umfaßt.

11. Verwendung des Verfahrens nach einem der Ansprüche 1 bis 7 zum Nachweis der Anwesenheit von Nukleinsäuresequenzen, die speziell mit Krankheiten, wie genetischen Erkrankungen oder Krebserkrankungen, Infektionen, assoziiert sind, oder geeignet sind, menschliche, tierische oder pflanzliche Individuen zu identifizieren.

**Claims**

1. Process for detecting and/or identifying a specific nucleotide base present on a target nucleic acid sequence of the type comprising an hybridisation step of the target sequence on which the base to be identified is present with a nucleotide sequence probe of sufficient length to permit a correct hybridisation, whatever the reaction temperature, a step of synthesis of the complementary strand of the obtained hybrid -the said nucleotide sequence probe serving as primer-, in the presence of a polymerase without 3'5' exonuclease action, and a step of detection of the base to be identified by any appropriate means, said process being characterised in that:

- said nucleotide sequence serving as primer, hybridises with the target sequence in such a way that its 3' end is adjacent to the specific nucleotide base to be detected and/or to be identified; and
- the synthesis of said complementary strand is performed in the presence of at least one blocking nucleotide base selected among dideoxynucleotides and in the absence of dNTP, said blocking nucleotide base, when it is incorporated, is detected and permits identification of the complementary specific nucleotide base present on the target sequence to be analysed.

2. Process according to claim 1, characterised in that said blocking nucleotide bases are labelled in an appropriate manner, in particular using a labeller chosen from the group which comprises radioactive substances, enzymes, chromophore fluorescent or chemiluminescent chemical products and appropriate antibodies.

3. Process according to claim 2, characterised in that the labeller is identical or different for each of the blocking nucleotide bases.

4. Process according to claim 3, characterised in that when the four blocking bases are labelled using different labellers, the detection of the four blocking nucleotides is advantageously simultaneous.

5. Process according to any one of claims 1 to 3, characterised in that when the four blocking bases are labelled in an identical manner or when they are not labelled, the detection of the four blocking nucleotides is carried out successively and/or separately.

6. Process according to claim 5, characterised in that the pyrophosphate formed during the polymerisation reaction is determined in an appropriate manner, the said pyrophosphate determination making it possible to determine in which one of the bases a polymerisation reaction is produced.

7. Process according to claim 5, characterised in that each labelled blocking nucleotide base is detected.

8. Ready-to-use diagnostic kit or box for carrying out the process according to any one of claims 1 to 7, characterised in that it comprises, in addition to the suitable amounts of reagents and buffers suited for carrying out the process:

   - appropriate amounts of a nucleotide, serving as primer, capable of hybridising with the target sequence in such a way that its 3' end is adjacent to the specific nucleotide base to be detected;
   - appropriate amounts of four blocking nucleotide bases selected among dideoxynucleotides in such a way that they can be incorporated in the extension product of the primer while blocking the elongation of the said extension product; and
   - appropriate amounts of a polymerase without 3'5' exonuclease action.

9. Kit or box according to claim 8, characterised in that the blocking nucleotide bases are labelled in an appropriate manner, in particular using a labeller chosen from the group which comprises radioactive substances, enzymes, chromophore fluorescent or chemiluminescent chemical products and appropriate antibodies.

10. Kit or box according to claim 8 or to claim 9, characterised in that it also comprises reagents suitable for the determination of the pyrophosphate.

11. Application of the process according to any one of claims 1 to 7 to the detection of the presence of particular nucleic acid sequences associated with diseases, such as genetic diseases or cancerous diseases, or with infections, or suitable for permitting the identification of human, animal or vegetable individuals.

FIG.1

FIG. 2